# FASCICULE DE BREVET EUROPEEN

(11) **EP 3 946 405 B1**
(45) Date de publication et mention de la délivrance du brevet: **07.06.2023**
(21) Numéro de dépôt: 20716942.6
(22) Date de dépôt: 23.03.2020
(51) Int. Cl.: A61K 36/48, A61K 31/455, A61P 27/02, A61K 9/00, A61K 31/555, A61K 45/06, A61K 33/24

(54) **COMPOSITION DE DESMODIUM ET DE CHROME TRIVALENT ET UTILISATION À VISEE OCULAIRE**
ZUSAMMENSETZUNG AUS DESMODIUM UND DREIWERTIGEM CHROM UND DEREN VERWENDUNG FÜR DIE AUGEN
COMPOSITION OF DESMODIUM AND TRIVALENT CHROMIUM, AND OCULAR USE

(30) Priorité: 25.03.2019 FR 1903045
(43) Date de publication de la demande: 09.02.2022
(73) Titulaire: Hvostoff, Sophie, 91540 Mennecy (FR); Yazbeck, Rima, 31520 Ramonville Saint Agne (FR)
(72) Inventeur: HVOSTOFF, Sophie, 91540 Mennecy (FR); YAZBECK, Rima, 31520 Ramonville Saint Agne (FR)
(74) Mandataire: Argyma
(86) Numéro de dépôt international: PCT/IB2020/052694
(87) Numéro de publication internationale: WO 2020/194166

(56) Documents cités:
- EP-A1- 3 273 947
- SUBHA RASTOGI ET AL: "An ethnomedicinal, phytochemical and pharmacological profile of(L.) DC. and(Sw.) DC", JOURNAL OF ETHNOPHARMACOLOGY, ELSEVIER IRELAND LTD, IE, vol. 136, no. 2, 12 avril 2011 (2011-04-12), pages 283-296, XP028375259, ISSN: 0378-8741, DOI: 10.1016/J.JEP.2011.04.031 [extrait le 2011-04-20]
- Sandip Buddhadev: "PHARMACOLOGICAL ACTIVITIES OF DESMODIUM GANGETICUM AN OVERVIEW View project", , 1 octobre 2013 (2013-10-01), XP055662430, Extrait de l'Internet: URL:https://www.researchgate.net/profile/S andip_Buddhadev/publication/318308121_PHAR MACOLOGICAL_ACTIVITIES_OF_DESMODIUM_GANGET ICUM_AN_OVERVIEW/links/5962117daca2728c11f d4f53/PHARMACOLOGICAL-ACTIVITIES-OF-DESMOD IUM-GANGETICUM-AN-OVERVIEW.pdf [extrait le 2020-01-28]
- AGE-RELATED EYE DISEASE STUDY RESEARCH GROUP: "A RANDOMIZED, PLACEBO-CONTROLLED, CLINICAL TRIAL OF HIGH-DOSE SUPPLEMENTATION WITH VITAMINS C AND E, BETA CAROTENE AND ZINC FOR AGE-RELATED MACULAR DEGENERATION AND VISION LOSS", ARCHIVES OF OPHTHALMOLOGY, AMERICAN MEDICAL ASSOCIATION, UNITED STATES, vol. 119, no. 10, 1 octobre 2001 (2001-10-01), pages 1417-1436, XP001098778, ISSN: 0003-9950

## Description

La présente invention concerne une composition comprenant une préparation de Desmodium et un sel de chrome trivalent, ainsi que son utilisation pour le traitement d'affections oculaires, notamment pour prévenir la formation et le développement des drusen précurseurs de la DMLA.

La DMLA (Dégénérescence Maculaire Liée à l'Age) correspond à une dégradation d'une partie de la rétine (la macula) pouvant conduire à la perte de la vision centrale. C'est la principale cause de malvoyance dans les pays développés chez les personnes de plus de cinquante ans. En Europe d'ici 2040, la DMLA précoce devrait affecter 14,9 millions à 21,5 millions de personnes, et entre 3,9 millions et 4,8 millions pour la forme avancée (Ophthalmology, 2017, Washington sl/ab/APMnews)*.*

La maladie débute par une phase sans dégénérescence, appelée maculopathie liée à l'âge (MLA) ou phase sèche précoce. La MLA peut rester stable durant une longue période de la vie, mais dans la moitié des cas et sous l'influence de divers facteurs, elle évolue vers une des formes dégénératives tardives : la forme atrophique dite DMLA sèche avancée, ou la forme humide, dite exsudative. Elles conduisent l'une comme l'autre à une dégradation irréversible de la macula et à une perte de la vision centrale affectant un seul oeil ou les deux. Il n'existe pas actuellement de traitement pour la DMLA forme sèche et/ou atrophique. Quant à la forme humide, le processus de dégénérescence peut être ralenti par des injections d'anticorps monoclonaux, sans toutefois empêcher l'évolution fréquente vers la forme atrophique incurable pour l'instant.

Dans sa phase précoce, la MLA se caractérise par l'accumulation de petits dépôts blanchâtres à l'intérieur et autour de la macula. Ces dépôts, aussi appelés druses ou drusen, sont visibles lors d'un simple examen de fond d'oeil. Processus normal du vieillissement oculaire, ils deviennent plus abondants avec un phénomène inflammatoire conduisant à une destruction accentuée de la rétine chez les patients présentant une DMLA.

De nombreuses études ont été menées et continuent de l'être pour comprendre les mécanismes à l'oeuvre dans les dysfonctionnements de la rétine et trouver des moyens d'y remédier. Une hypothèse considère qu'avec le vieillissement, les cellules de l'épithélium pigmentaire rétinien se détériorent, entrainant un apport insuffisant d'oxygène aux cellules photoréceptrices qui ne peuvent plus envoyer de signaux visuels au cerveau. Ceci provoque une moindre destruction des déchets qui s'accumulent au niveau de l'œil. Cette accumulation entraine le dépôt de drusen, précurseurs de la DMLA. Une autre hypothèse est que les vaisseaux sanguins sous-jacents qui fournissent les nutriments et rejettent les déchets de l'épithélium pigmentaire rétinien et des photorécepteurs, ne fonctionnent pas bien. La conséquence est un manque d'oxygène et une accumulation des déchets. L'hypoxie provoque la libération d'un facteur de croissance vasculaire qui à son tour induit un mécanisme de compensation par la formation de nouveaux vaisseaux sanguins anormaux.

En 2002, une équipe de chercheurs américaine a étudié les druses du fond d'oeil, d'yeux affectés de DMLA et d'yeux non malades. Ils ont trouvé des modifications des protéines au sein des druses de malades, avec des altérations qui peuvent être dues à l'oxydation des lipides et des sucres. Un lien moléculaire est ainsi établi entre les altérations oxydatives et la DMLA (Crabb J.W., Proc Natl Acad Sci USA, 2002, Early Edition 10.1073/pnas 222551899).

De multiples questions restent ouvertes concernant l'apparition et les cause d'évolution de la pathologie. À côté des théories génétiques évoquant des déficiences en enzymes épithéliaux, se sont développées des explications basées sur des carences nutritionnelles, notamment en vitamines antioxydantes. Les études menées séparément sur la vitamine C, la vitamine E, le caroténoïde ou le zinc ont donné des résultats controversés quant au lien avec la DMLA et l'intérêt d'une supplémentation.

L'étude AREDS (Age Related Eye Disease Study) a été la première étude randomisée et contrôlée concernant l'effet d'un apport de vitamines antioxydantes et de zinc administrés seuls ou ensemble, à des doses bien supérieures aux apports quotidiens recommandés (Arch. Ophthamol., 2001;119:1417-36)*.* L'étude a évalué l'effet protecteur d'une association d'antioxydants à hautes doses de vitamine C (500 mg/j), vitamine E (400 Ul/j), bêta-carotène (15 mg/j) et de zinc (80 mg/j). Cette association s'est avérée avoir un effet protecteur chez les patients ayant déjà perdu la vision centrale sur un oeil (stade 4 de la classification AREDS) et chez les patients atteints de larges drusen ou d'atrophie géographique sans atteinte centrale (stade 3 de la classification AREDS).

Les résultats obtenus après six années de suivi permettent ainsi d'affirmer le bénéfice d'une thérapeutique préventive par supplémentation alimentaire en vitamines antioxydantes et en zinc au stade intermédiaire de la DMLA. Par contre, aucune recommandation pour une telle supplémentation n'a pu être faite de manière formelle pour les patients présentant des formes précoces de MLA ou une DMLA modérée, avec des drusen de moins de 125 µm, correspondant aux catégories 1 et 2 de la classification de l'AREDS.

En outre, l'étude AREDS attire l'attention sur les risques d'une accumulation toxique de vitamine A au niveau de l'épithélium pigmentaire. Sa prescription à fortes doses est également suspectée d'aggraver l'accumulation toxique de produits du métabolisme favorisant l'atrophie (Cohen S. Y., Bull. Soc. belge Ophthalmol., 2006, 301, 33-36)*.*

Par ailleurs, le bêta-carotène présente un risque chez les patients fumeurs, ce qui a conduit l'industrie pharmaceutique à proposer d'autres cocktails dans lesquels il est remplacé par d'autres pigments, principalement par la lutéine et la zéaxanthine ou la méso-zéaxanthine. Cependant, aucune étude randomisée et contrôlée ne permet d'affirmer l'efficacité de tels cocktails (*ibid.*)*.* Par la suite, d'autres molécules ont été introduites dans les cocktails destinés à une complémentation alimentaire à visée oculaire, tels que des acides gras de type oméga-3, particulièrement le DHA (acide docosahexaénoïque). Cependant aucun complément alimentaire actuellement disponible n'est complètement satisfaisant.

Il existe donc un besoin de mettre au point des compositions efficaces, de manière à proposer un traitement efficace des maculopathies à un stade précoce pour soulager les patients gênés par le développement de drusen, permettant d'éviter une aggravation des symptômes vers des formes dégénératives de la DMLA, et ne présentant pas de risques associés à leur prise ni d'effets indésirables sur certains groupes de personnes.

Il a été découvert de manière surprenante que l'association du Desmodium avec un sel de chrome trivalent permet d'obtenir une action efficace sur les drusen, aboutissant à leur régression, voire à leur disparition.

La présente invention a donc pour objet une composition comprenant une préparation de Desmodium et un sel de chrome trivalent.

Le Desmodium est une plante herbacée faisant partie des Fabacée dont l'aire géographique va de la zone équatoriale aux régions humides de la zone tropicale de l'Afrique de l'ouest. Environ 300 espèces du genre Desmodium existent dans le monde et 25 espèces différentes sont utilisées dans les médecines traditionnelles à travers le monde. Les plus utilisées sont inscrites à la pharmacopée Indienne et chinoise, comme *D*. *Girans, D. Latifolium, D. Polycarpum, D. Pullchellium, D. Tiliaffolium, D. Trifiorum, D. Styracifolium, D. Incanum, D. Canadense, D. Sessilifolium, D. Illinoensis.* Doivent y être ajoutées *Desmodium adscendens* et *Desmodium gangeticum* dont de nombreuses activités pharmacologiques ont été décrites, notamment des propriétés hépatoprotectrices, immunomodulatrices, antiasthmatiques, myorelaxantes, anti-ulcères, anti-inflammatoires, cardio-protectrices (Rastogi S. et coll., Journal of Ethnopharmacology, 2011, 136-2, 283-296). À Madagascar, un laboratoire a obtenu en 2013 un agrément du Ministère de la Santé pour la production de *Desmodium adscendens* et le commercialise dans un phyto-médicament qui a reçu une autorisation de mise sur le marché (AMM).

Peu de composants ont été isolés de *Desmodium adscendens.* Les composants qui ont été identifiés en premier lieu dans ses feuilles en milieu aqueux, sont des saponines triterpénones, des tétrahydro-isoquinolones, des dérivés de la tryptamine (phényléthyl-amines et indole-3-alkylamines) (Addy M.E., Internal Journal of Crude Drugb Research 1989; 27:81-91). Par la suite, parmi les saponines, trois composants très étudiés en pharmacologie pour leur action agoniste sur certains canaux calciques, ont été isolés : la déhydrosoyasaponine I (DHS1), composé majoritaire, et les soyasaponines I et III *(*Mc Manus O.B. et coll., Biochemistry, 1993; 32 :6128-33*)*. On a aussi isolé de la tyramine et de l'hordénine (Addy M.E. et coll., Phytotherapy Research, 1992; 6 :245-50*).* Plus récemment, un sucre a été identifié dans les feuilles et les tiges, le d-pinitol (Malgielse J. et coll., Journal of Ethnopharmacology, 2013 ; 146: 250-56*).* Des travaux sur les flavonoïdes ont identifié la présence de vitexine et d'isovitexine (hétérosides de flavones) (Heard O., 1994, Thèse d'état pour le doctorat en pharmacie, faculté de Tours*).*

*Desmodium adscendens* est le plus souvent cité mais *Desmodium gangeticum* pourrait lui être substitué. Dans un mode de réalisation de la composition selon l'invention, la préparation de Desmodium est une poudre *Desmodium adscendens* ou de *Desmodium gangeticum* obtenue par broyage des feuilles, des tiges ou des parties aériennes de la plante. Cette poudre est facile à conserver et pourra être introduite ultérieurement dans des formulations comprenant d'autres actifs et des excipients, sous des formes galéniques sèche, pâteuse ou en phase liquide.

Le chrome trivalent (Cr III), de masse atomique 52, est nécessaire à l'utilisation des acides gras et des protéines, ainsi qu'à celle des glucides. C'est un potentialisateur de l'insuline et comme cofacteur de l'insuline, il intervient également sur le métabolisme lipidique. Une carence peut provoquer des problèmes cardiaques, des perturbations du métabolisme et du diabète, tandis qu'une absorption excessive peut être à l'origine de problèmes tels que des éruptions cutanées. Chez l'Homme, les apports nutritionnels conseillés (ANC) sont de 40 µg/j selon la Directive 2008/100/CE. Le chrome III peut être apporté sous la forme d'un sel, en association avec un contre-ion comme le chlorure, le nicotinate ou le picolinate. Il est également proposé sous forme de levure de bière enrichie.

Selon un mode de réalisation de la composition selon l'invention, le sel de chrome est choisi parmi le chlorure de chrome, le nicotinate de chrome, le picolinate de chrome. Le picolinate de chrome et le nicotinate de chrome (ou respectivement chrome (III) picolinate et chrome (III) nicotinate selon la nomenclature de l'UICPA) sont des composés isomères de formule Cr(C₆H₄NO₂)₃ ayant une masse molaire moyenne de 418,33 g. Le chlorure de chrome est avantageusement utilisé sous forme de solution, proposée en ampoule dose par exemple.

Selon un mode de réalisation de l'invention, la composition comprend de 0,2 mg à 1,2 mg de chrome III par gramme de Desmodium. De préférence, elle comprend 0,25 mg à 0,5 mg de chrome III par gramme de Desmodium, et encore de préférence 0,33 mg par gramme de Desmodium.

La composition selon l'invention peut se présenter pure, c'est-à-dire ne contenant que ses composants essentiels, à savoir du Desmodium et du sel de chrome, mais peut également inclure d'autres composés ayant une action complémentaire avec les composants essentiels. Ainsi, la composition peut comprendre de 30% à 100% de préparation de Desmodium et de sel de chrome, en masse par rapport à la masse totale de la composition.

Parmi les ingrédients susceptibles d'accompagner l'association Desmodium-Cr III, on trouve des vitamines, et en particulier des vitamines à effet antioxydant, telles que l'acide ascorbique (C), la vitamine E, la riboflavine (B2), la niacine (B3), la pyridoxine (B6), l'acide folique (B9) ou la cobalamine (B12). D'autres vitamines peuvent être aussi incorporées à la composition, comme la choline (B4), la thiamine (B1), la biotine (B8). Ainsi, dans un mode de réalisation, la composition selon l'invention comprend au moins une vitamine choisie parmi la vitamine C, la vitamine E, la vitamine B1, la vitamine B2, la vitamine B3, la vitamine B4, la vitamine B6, la vitamine B8, la vitamine B9 et la vitamine B12.

Selon un autre mode de réalisation de l'invention, la composition peut comprendre au moins un pigment choisi parmi la lutéine et la zéaxanthine. Ces pigments de couleur jaune-orangé appartiennent à la famille des caroténoïdes filtrant la lumière dans les végétaux (sans toutefois présenter d'activité provitaminique A), et sont présents en très forte concentration dans la rétine. Ils ont des propriétés antioxydantes et sont proposés pour leur rôle protecteur des tissus fragiles contre l'agression de la lumière solaire.

La composition objet de l'invention peut inclure également des ingrédients riches en acides gras de type oméga-3, et en particulier en DHA (acide docosahexaénoïque), en EPA (acide eicosapentaénoïque) et/ou en ALA (acide alpha-linolénique). Le DHA intervient dans pratiquement tous les organes, c'est l'oméga-3 le plus abondant dans le cerveau et la rétine. Une étude ayant mis en garde sur les effets des oméga-3 issus des huiles de poissons utilisées dans les compléments alimentaires à visée oculaire (cancer de la prostate chez l'homme (BRASKY et coll., 2013, Journal of the National Cancer Institute), on privilégie les sources végétales, en particulier les microalgues telles que *Schizochytrium* ou *Odontella aurita,* contenant des oméga-3 en grande quantité. Ces microalgues présentent l'intérêt d'être en même temps riches en pigments xanthophylles (notamment en lutéine). D'un point de vue nutritionnel, *Odontella aurita* présente un profil de composés particulièrement intéressants, d'autant que c'est la première microalgue marine à avoir été autorisée en nutrition humaine. Elle produit des acides gras polyinsaturés à longues chaînes dont principalement de l'EPA et du DHA, molécules indispensables pour le développement du système nerveux central, de la vision et de la protection du système cardiovasculaire (EFSA, 2011). En outre, chez *Odontella aurita,* le rapport oméga-3/oméga-6 est d'environ 8, ce qui contribue à compenser le déséquilibre des aliments industriels trop riches en oméga-6 (rapport oméga-3/oméga-6 de 0,03 à 0,10). Elle est commercialisée sous forme de poudre ou d'huile. *Schizochytrium,* également d'origine marine, contient de grandes quantités de DHA. Elle est cultivée pour la production de DHA, cette huile étant destinée notamment à la consommation humaine dans des compléments et additifs alimentaires. Les microalgues capables de produire des quantités significatives de DHA les plus souvent citées sont celles appartenant aux genres *Schizochytrium, Odontella, Crypthecodinium, Phaeodactylum et Ulkenia.* De nombreuses autres plantes constituent aussi des sources appréciées d'oméga-3. On peut utiliser par exemple des graines de lin, ou de l'huile de noix, de l'huile de colza, des noix sèches, du cresson, du chou, des épinards ..., que l'homme du métier connaît pour ce type d'applications. Ces composants peuvent être apportés sous la forme d'un broyat sec de microalgue, ou bien après extraction, sous forme d'une huile ou d'une pâte grasse.

Dans une variante de réalisation, la composition selon la présente invention comprend une source d'acides gras de type oméga-3. De préférence, la source d'oméga-3 est choisie parmi une préparation de *Schizochytrium,* une préparation *d'Odontella aurita,* une préparation de graines de lin, ou un mélange de celles-ci.

Selon un mode de réalisation, la composition selon l'invention comprend de 30% à 75% de Desmodium et de sel de chrome, de 10% à 30% de vitamines, et de 0,5% à 2,5% de lutéine et zéaxanthine, et de 25 % à 60% d'une préparation d'huile de *Schizochytrium* ou *d'Odontella,* en masse par rapport à la masse totale de la composition.

Selon un mode de réalisation préféré, la composition selon l'invention comprend de 60% à 65% de Desmodium et sel de chrome, de 15% à 18% de vitamines, de 0,75% à 1,5% de lutéine et zéaxanthine, et de 30 % à 40% d'une préparation d'huile de *Schizochytrium* ou d'*Odontella,* en masse par rapport à la masse totale de la composition.

Selon une caractéristique intéressante, la composition selon l'invention comprend en outre une préparation sèche de d'*Aphanizomenon flos-aquae* (ou plus simplement AFA) ou de Spiruline. L'AFA est une algue bleu vert aussi appelée Algue Super Blue Green, qui est originaire du Lac Klamath dans l'ouest des États-Unis. Sa composition est riche en micronutriments, dont le pigment bleu phycocyanine, et en acides gras polyinsaturé oméga-3 en font un complément fréquemment utilisé dans les compléments alimentaires. Elle est généralement préparée par un séchage doux protégeant ses constituants et puis réduite en poudre. La Spiruline est également appréciée pour sa haute teneur en micronutriments.

De manière optionnelle, la composition objet de l'invention peut contenir du zinc. Le zinc est présent à une concentration élevée dans la rétine, et joue un rôle très important dans les mécanismes de défense antioxydante. Selon l'étude AREDS déjà citée, aucun effet chez les patients atteints de drusen inférieur à 125 µm n'a été relevé, mais un effet protecteur dans la DMLA aux stades 3 et 4 est noté pour le zinc associé aux vitamines, aidant à une meilleure réduction des lésions maculaires.

La plupart des composants de la composition objet de l'invention peuvent se présenter à l'état solide, généralement sous la forme de poudres fines qu'il est facile de préparer, de stabiliser pour les conserver et de stocker. On peut donc faire aisément le choix d'une formulation particulière et procéder à un mélange qui sera lui-même à l'état solide et facilement conservé. Selon la formulation choisie la composition peut être "pure", c'est-à-dire qu'elle ne contient que les actifs retenus. Cependant, certains composants ne sont eux-mêmes pas purs, mais obtenus et commercialisés dans une phase support. Lors de la fabrication de la composition, ces supports se retrouveront donc dans le mélange. Il est aussi possible qu'un excipient ou un additif doive être introduit dans ce mélange, tel qu'un agent liant (par exemple de la cellulose microcristalline), un agent antiagglomérant (par exemple du stéarate de magnésium), un colorant, ou autre excipient pharmaceutique acceptable bien connu de l'homme du métier comme apte à entrer dans ce type de composition. Il est entendu à ce sujet que, sauf indication contraire, dans la présente description, les composés sont mentionnés en tant que produits actifs, sans prendre en compte ni les éventuels additifs et excipient, ni la dilution dans une phase support.

Quoi qu'il en soit, tous les additifs et excipients utilisés dans la composition sont adaptés à un usage alimentaire ou pharmaceutique oral et sont autant que possible choisis parmi des produits d'origine végétale. Le mélange en poudre peut ensuite être administré en l'état, le patient prélevant une dose à l'aide d'une cuillère graduée ou d'un autre moyen à sa disposition. Le mélange peut aussi être mis sous une forme galénique unitaire pré-dosée, telle que comprimés, gélules, capsules molles ou autre. La forme galénique en gélules utilise de préférence une enveloppe végétale, par exemple en hydroxypropylméthyl-cellulose (HPMC), de 70 mg à 75 mg.

Ainsi, selon un mode de réalisation, la composition est formulée seule ou avec au moins un excipient ou additif, pour être administrée par voie orale sous forme de gélules, de comprimés, de capsules molles, ou encore sous forme liquide, en ampoules-doses ou en flacon. Il est précisé que tous les ingrédients constituant la composition ne sont pas obligatoirement formulés en un seul et unique emballage-dose, mais peuvent être répartis en deux emballages-doses (ou davantage) qui seront pris de manière concomitante ou répartis dans la journée. Par exemple, une partie des composants peut être contenue dans une gélule, et les composants restant dans une seconde gélule, ou dans un comprimé, ou encore se trouver à l'état liquide, dans un flacon, une ampoule, ... Le principe est que les différents composants soient administrés à court intervalle de temps les uns des autres, de sorte qu'ils agissent simultanément.

Selon un mode de réalisation avantageux, la composition objet de l'invention la composition est administrée de manière quotidienne, sous la forme d'un ou de deux emballages-doses. Par exemple, elle peut être administrée de manière à apporter une dose journalière de 0,2 g à 1,0 g de Desmodium et de chrome III, étant entendu que le Desmodium et le chrome III sont dans les proportions relatives définies plus haut. De préférence, on peut apporter une dose journalière de 0,5 g à 0,7 g de Desmodium et de 0,19 mg à 0,21 mg de chrome III. La dose journalière peut être administrée en une prise, ou répartie en deux ou trois prises réparties dans la journée.

La composition qui vient d'être décrite peut être prescrite en tant que médicament dans le cadre du traitement thérapeutique de troubles visuels liés à l'apparition de drusen sur la rétine. Un tel médicament peut aussi être administré à titre préventif, afin d'anticiper les phénomènes risquant d'aggraver des problèmes de santé ou d'entrainer leur apparition. Plus particulièrement, il peut être considéré comme un phyto-médicament, les phyto-médicaments étant des médicaments dont le principe actif provient d'une plante entière ou d'une partie de cette plante. Selon certaines réglementations, il peut être assimilé à un dispositif médical. Également, la composition peut être considérée comme un complément alimentaire à visée oculaire, et préconisé comme tel. Dans certains cas, il sera intéressant de l'inclure dans un produit alimentaire, de sorte que la prise se fasse sans effort tout en respectant les recommandations diététiques pour la prévention de la DMLA.

Ainsi, la composition objet de la présente invention peut être utilisée en tant que médicament, phyto-médicament, dispositif médical, complément alimentaire ou mélangée à un produit alimentaire.

Elle est ainsi destinée à être utilisée pour la prévention ou le traitement d'une pathologie dégénérative de la rétine choisie parmi la maculopathie liée à l'âge (MLA), la dégénérescence maculaire liée à l'âge (DMLA) sèche et la dégénérescence maculaire liée à l'âge exsudative. En particulier, elle peut être prescrite pour prévenir l'apparition de drusen, et le cas échéant, obtenir la diminution ou l'élimination des drusen. Dans le traitement de la DMLA, sont visées les formes sèches précoces comme les formes sèches avancées, ainsi que les DMLA humides (ou exsudatives).

Elle peut être prescrite pour des durées prolongées autant que nécessaire, en fonction des résultats des examens de contrôle (OCT). Il est couramment préconisé une prise selon des cycles de 90 jours, avec 60 jours de traitement interrompus par une fenêtre thérapeutique de 30 jours.

De manière inattendue, dans la composition telle que décrite ci-dessus, un effet synergique se produit entre le Desmodium et le sel de chrome III, c'est-à-dire qu'on observe un effet supérieur de la combinaison de Desmodium et de chrome III en comparaison aux effets additionnés que l'on peut observer lors de la prise de compositions identiques ne comprenant chacune que le Desmodium ou que le sel de chrome. Suite au suivi d'un traitement conforme à l'invention, on a pu observer une amélioration significative de l'acuité visuelle. Les examens réalisés par tomographie par cohérence optique (OCT) ont mis en évidence une réduction du nombre de drusen séreux (de hauteur supérieure à 125 µm), indice conditionnant le pronostic visuel dans la DMLA.

La présente invention sera mieux comprise, et des détails en relevant apparaîtront, à la lumière de la description qui va être faite de différentes variantes de réalisation, en relation avec les figures annexées, dans lesquelles :
Les figures 1a et 1b sont des images obtenues par OCT de l'œil droit d'un patient, avant et après traitement par une composition selon l'invention.
Les figures 2a et 2b sont des images obtenues par OCT de l'œil gauche du même patient, avant et après traitement par une composition selon l'invention.

### EXEMPLE 1 : Liste des ingrédients

Les ingrédients ci-dessous peuvent être utilisés pour préparer une composition selon l'invention, dans les proportions suivantes. Les quantités indiquées correspondent à des doses journalières. On note que certains dosages sont particulièrement élevés, ce qui est adapté pour la DMLA (conformément à l'étude AREDS).
*Desmodium adscendens* : entre 400 à 800 mg
Sel de chrome :
   chlorure de chrome compris entre 0,10 mg et 0,25 mg, ou
   nicotinate de chrome compris entre 0,10 mg à 0,25 mg, ou
   picolinate de chrome compris entre 0,10 mg à 0,25 mg
Lutéine : entre 5 mg et 10 mg
Zéaxanthine : entre 1 mg et 2 mg
Zinc : entre 7 mg et 80 mg
Cuivre : entre 0,5 mg et 2 mg
Oméga-3 sous forme d'huile de poisson ou microalgues ou végétaux dont
DHA : entre 180 mg et 502 mg (acide docosahexaènoïque)
DPA : 100 mg (acide docosapentaènoïque)
EPA : 650 mg (acide eicosapentaènoique)
Vitamine C : entre 120 mg et 500 mg
Vitamine E : entre 30 mg et 40 mg
Vitamine B1 : entre 0,50 mg et 1,10 mg
Vitamine B2 : entre 0,70 mg et 1,40 mg
Vitamine B3 : entre 8 mg et 16 mg
Vitamine B4 : entre 400 mg et 500 mg
Vitamine B6 : entre 0,70 mg et 1,40 mg
Vitamine B8 : entre 0,025 mg et 0,035 mg
Vitamine B9 : entre 0,1 mg et 0,2 mg
Vitamine B9 : entre 0,1 mg et 0,2 mg
Vitamine B12 : entre 0,00125 mg et 0,0025 mg

### EXEMPLE 2 : FORMULATIONS

Les quantités indiquées correspondent à des doses journalières.

### Formulation 1 :

| | |
|---|---|
| *Desmodium adscendens* | 600 mg, feuilles |
| Nicotinate de Chrome | 0,2 mg (masse du chrome apporté) |

Cette composition solide peut être administrée en 1 ou 5 gélules-doses par jour.

### Formulation 2 :

| | |
|---|---|
| *Desmodium adscendens* | 600 mg, feuilles et tiges |
| Chlorure de Chrome | 0,2 mg (masse du chrome apporté) |

Cette composition peut se présenter sous forme liquide en ampoule ou en flacon. Elle peut aussi se présenter en deux emballages unitaires, l'un en gélule contenant le *Desmodium adscendens* en poudre, l'autre en ampoule contenant le chlorure de chrome en solution. Elle peut être administrée en 1 ou 2 prises journalières.

### Formulation 3 :

| | |
|---|---|
| *Desmodium adscendens* | 600 mg, feuilles |
| Picolinate de de Chrome | 0,2 mg (masse du chrome apporté) |

Cette composition solide peut être administrée en 1 ou 5 gélules-doses par jour.

### Formulation 4 :

| | |
|---|---|
| *Desmodium adscendens* | 400 mg |
| Chlorure de Chrome | 0,1 mg (masse du chrome apporté) |
| Lutéine | 5 mg |
| Zéaxanthine | 1 mg |
| Oméga-3 | 300 mg dont DHA 180 mg |
| Zinc | 7,5 mg |
| Vitamine C | 90 mg |
| Vitamine E | 15 mg |

Cette composition peut être répartie en 3 gélules et 1 ampoule administrées en 1 à 2 prises réparties dans la journée.

### Formulation 5 :

| | |
|---|---|
| *Desmodium adscendens* | 600 mg |
| Nicotinate de Chrome | 0,2 mg (masse du chrome apporté) |
| Lutéine | 5 mg |
| Zéaxanthine | 1 mg |
| Oméga-3 | 338 mg dont DHA 251,5 mg, EPA 56,50 mg, DPA 37,50 mg |
| Zinc | 7,5 mg |
| Cuivre | 0,5 mg |
| Vitamine C | 90 mg |
| Vitamine E | 15 mg |
| Resvératrol | 0,50 mg |

Cette composition solide peut être répartie en 6 gélules administrées en 1 ou 2 prises réparties dans la journée.

### Formulation 6 :

| | |
|---|---|
| *Desmodium adscendens* | 600 mg |
| Picolinate de Chrome | 0,20 mg (masse du chrome apporté) |
| Lutéine | 5 mg |
| Zéaxanthine | 1 mg |
| Oméga-3 | 185 mg de DHA |
| Vitamine C | 60 mg |
| Vitamine E | 15 mg |
| Vitamine B1 | 0,55 mg |
| Vitamine B2 | 0,7 mg |
| Vitamine B3 | 8 mg |
| Vitamine B6 | 0,7 mg |
| Vitamine B8 | 0,025 mg |
| Vitamine B9 | 0,1 mg |
| Vitamine B12 | 0,00125 mg |

Cette composition solide peut être répartie en 6 gélules administrées en 2 ou 3 prises réparties dans la journée.

### Formulation 7 :

| | |
|---|---|
| *Desmodium adscendens* | 600 mg poudre ou équivalent buvable |
| Chlorure de Chrome | 0,20 mg (masse du chrome apporté) |
| Lutéine | 10 mg |
| Zéaxanthine | 2 mg |
| Zinc | 7,5 mg |
| *Schizochytrium* | 500 mg d'oméga-3 |
| dont DHA 32%±2 soit | 160-170 mg et DPA 4%± 2 soit 20-30 mg |
| *Aphanizomenon flos Aquae* | 20 mg |
| Vitamine C | 120 mg |
| Vitamine E | 30 mg |
| Vitamine B1 | 0,55 mg |
| Vitamine B2 | 1 mg |
| Vitamine B3 | 8 mg |
| Vitamine B4 | 400 mg |
| Vitamine B6 | 1 mg |
| Vitamine B8 | 0,025 mg |
| Vitamine B9 | 0,1 mg |
| Vitamine B12 | 0,0025 mg |

### Formulation 8 :

| | |
|---|---|
| *Desmodium adscendens* | 600 mg poudre ou équivalent buvable |
| Nicotinate de Chrome | 0,20 mg (masse du chrome apporté) |
| Lutéine | 10 mg |
| Zéaxanthine | 2 mg |
| Zinc | 7,5 mg |
| *Odontella* | 500 mg d'Oméga-3 |
| dont DHA 32%±2 soit | 160-170 mg et DPA 4%± 2 soit 20-30 mg |
| Spiruline | 20 mg |
| Vitamine C | 120 mg |
| Vitamine E | 30 mg |
| Vitamine B1 | 0,55 mg |
| Vitamine B2 | 1 mg |
| Vitamine B3 | 8 mg |
| Vitamine B4 | 400 mg |
| Vitamine B6 | 1 mg |
| Vitamine B8 | 0,025 mg |
| Vitamine B9 | 0,1 mg |
| Vitamine B12 | 0,0025 mg |

### EXEMPLE 3 : TESTS

| | |
|---|---|
| Abréviations utilisées : - OD : oeil droit | - OG : oeil gauche |
| - OCT : Optical Cohérence Tomography pour tomographie en cohérence optique | |

### PERIODE N° 1

État initial : Présence depuis plusieurs années d'un remaniement maculaire avec altération de l'épithélium gauche et une discrète opacification du cristallin à l'œil droit.

Traitement : Complément alimentaire CA1 à visée oculaire de type connu à raison de 2 gélules/j.

Contenu d'1 gélule du complément alimentaire CA1 (nom commercial Préservision3^{®}) :

| | |
|---|---|
| - oméga-3 | 300 mg (dont DHA 180 mg) |
| - Lutéine | 5 mg |
| - Zéaxanthine | 1 mg |
| - Vitamine C | 90 mg |
| - Vitamine E | 15 mg |
| - Zinc | 7,5 mg |

Effet / résultat : Malgré ce traitement, une baisse brutale de la vision de loin s'est produite. L'acuité visuelle est passée en quelques mois, de 0,8 à 0,6 (OD et OG).

### PERIODE N° 2

État initial : La consultation ophtalmologique indique que l'acuité visuelle est descendue à 0,6 (OD et OG). L'examen du fond de l'œil droit montre un remaniement pigmentaire de la macula et une suspicion de drusen à l'œil gauche.

Traitement : Complément alimentaire CA1 à raison d'1 gélule/j et chlorure de chrome à raison de 100 µg/j (solution aqueuse buvable).

Effet / résultat : Après 6 mois, l'acuité visuelle est stable pour les deux yeux ODG 0.6. L'examen montre un remaniement pigmentaire de la macula. Le diagnostic d'une DMLA sèche ODG est posé, avec présence de plusieurs drusen confluents. Cependant, au bout d'un an, l'acuité visuelle s'est fortement dégradée (0,3 OG et 0,6 OD). L'examen OCT montre des drusen séreux (dont la hauteur est ≥125 µm) en nombre important : 6 drusen à l'œil droit (hauteurs mesurées 122, 125, 140, 176, 214 et 197) et 6 drusen à l'œil gauche (hauteurs mesurées 128, 131, 169, 125, 124 et 123). Néanmoins, il n'y a pas d'exsudation intra-rétinienne (pas de néovaisseaux), ce qui indique qu'il n'y a pas à ce stade d'évolution vers une DMLA humide avec néo vaisseaux. Le complément alimentaire CA1 et le sel de chrome associés n'ont pas permis d'améliorer l'état de la patiente.

### PERIODE N° 3

État initial : Acuité OD 0,6 ; OG 0,3. Présence de nombreux drusen séreux, pas d'exsudation intra-rétinienne.

Traitement : Le traitement est conforme à la Formulation 4 donnée à l'exemple 1 ci-dessus. Il correspond au traitement de la période précédente (CA1 1 gélule, et chlorure de chrome 100 µg par jour) auquel est ajouté du *Desmodium adscendens* 400 mg/j. Prise par cycles, pendant 1 mois puis 1 mois d'arrêt.

Effet / résultat : État maculaire stable, une légère amélioration est observée OG 0,25.

### PERIODE N° 4

État initial : Acuité OD 0,6 ; OG 0,25. Présence de drusen séreux, pas d'exsudation intra-rétinienne.

Traitement : Le traitement est modifié avec des doses quotidiennes suivantes : CA1 (1 gélule) et apport renforcé à 600 mg en *Desmodium adscendens* et à 200 µg en sel de chrome, pendant 2 mois puis 1 mois d'arrêt.

Effet / résultat : Au bout de six mois, l'examen OCT fait apparaitre une diminution des drusen, avec disparition des drusen séreux à l'œil gauche, l'œil droit portant toujours six druses. L'acuité visuelle a été mesurée à OD 6/10 et OG 4/10, montrant une nette amélioration. Le fond de l'œil montre une micro-atrophie pour les deux yeux. Ainsi, l'association Desmodium et chrome III a un effet sensible et rapide sur l'acuité visuelle et sur la diminution des drusen.

### PERIODE N° 5

État initial : Évolution favorable de la vision et régression des drusen.

Traitement : Compte tenu de l'amélioration obtenue précédemment, le traitement est maintenu, mais sans interruption d'un mois sur trois.

Effet / résultat : Après 5 mois de traitement en continu, le bilan biologique découvre une augmentation du chrome urinaire à 31,54 nmol/l. Il est donc préférable de réduire l'apport de chrome au long cours. Le traitement est modifié en réintroduisant un mois d'arrêt par trimestre. Après un trimestre, les analyses indiquent un début de diminution du taux de chrome urinaire (≤ 2 nmol/l).

### PERIODE N° 6

État initial : Acuité visuelle mesurée en amélioration par rapport au semestre précédent, à OD 6/10 et OG 4/10. Présence de chrome supérieure à la normale dans les urines. Traitement : L'association *Desmodium adscendens* 600 mg et chlorure de chrome 200 µg est maintenue et est incluse dans la Formulation 5, telle que donnée à l'exemple 1 ci-dessus., avec prise pendant 2 mois puis 1 mois d'arrêt.

Effet / résultat : En l'espace d'un an, l'acuité visuelle s'est fortement améliorée avec 9/10 OD et 9/10 OG, soit une amélioration de 3/10 (OD) et de 5/10 (OG). L'OCT montre une nette diminution des drusen séreux : 4 drusen persistent à l'œil droit (hauteurs mesurées 149, 137, 169 et 130) et aucun drusen de plus de 125 µm à l'œil gauche. Le contrôle du chrome sanguin indique un retour à la normale (≤ 0,52 µg/l). Ainsi, les drusen n'ont pas progressé, la Formulation 5 a permis de stabiliser la MLA et a amélioré la vision.

### PERIODE N° 7

État initial : Bon niveau d'acuité visuelle (0,9 à chaque oeil) et drusen stables.

Traitement : *Desmodium adscendens* 600 mg est associé avec 200 µg de chrome sous forme de picolinate. Ils sont inclus dans la Formulation 6, telle qu'à l'exemple 1 ci-dessus. La composition est administrée par cycles avec prise durant 1 mois et 1 mois d'arrêt.

Effet / résultat : On constate un maintien de l'acuité visuelle OD 7/10 et OG 8/10. L'examen OCT montre que les drusen ont encore régressé : une druse de 145 µm (OD) et une druse de 126 µm (OG). Le chrome sanguin est à un niveau normal. Une baisse de la vision à 0,7 et 0,8 pourrait provenir d'une opacification du cristallin (cataracte). La Formulation 6 permet d'obtenir des résultats satisfaisants en ce qui concerne la régression des drusen.

### PERIODE N° 8

Traitement : Un arrêt complet de tout traitement a été observé sur une période de 6 mois.

Effet / résultat : L'acuité visuelle est restée stable sur la période. Par contre, une légère augmentation des drusen séreux a été constatée, et surtout de nombreux petits drusen se sont formés aux deux yeux, laissant présager une reprise de l'évolution naturelle de la pathologie due à l'arrêt du traitement.

### PERIODE N° 9

État initial : Acuité visuelle OD 8/10 et OG 7/10.

Traitement : Le traitement basé sur la Formulation 5 donnée à l'exemple 1 est administré avec arrêt un mois par trimestre.

Effet / résultat : L'acuité visuelle reste stable (OD 7/10 et OG 7/10). L'examen OCT montre une diminution des drusen (un drusen de 140 µm OG et aucun drusen OD). La reprise du traitement s'est avérée nécessaire pour une nouvelle régression des drusen.

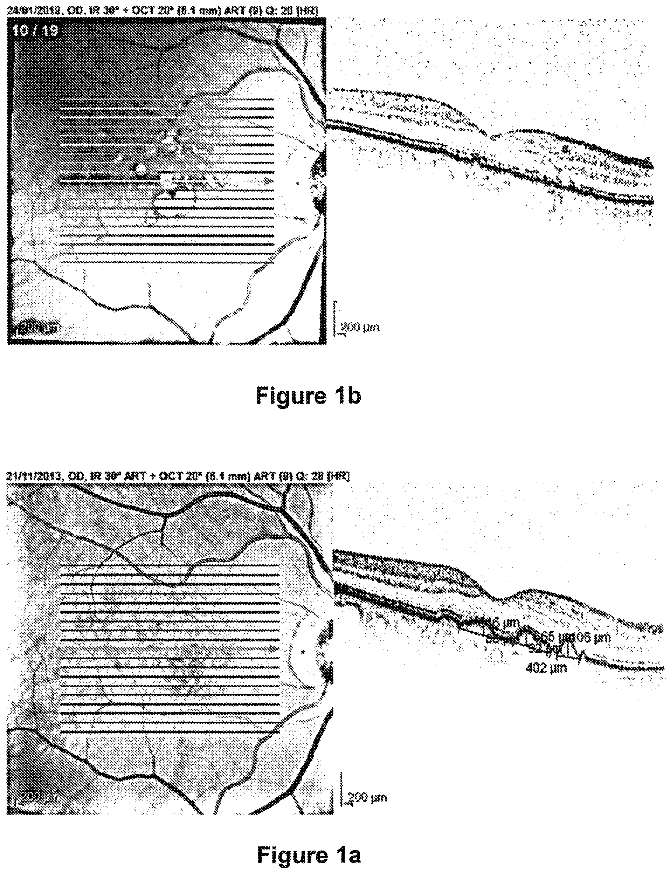

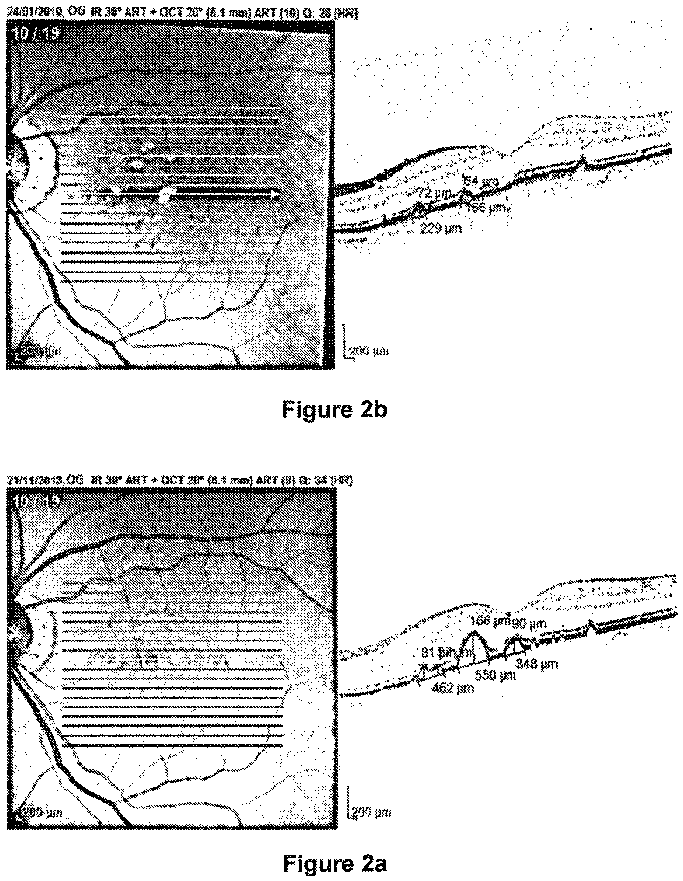

L'amélioration obtenue est illustrée par les images des examens OCT données aux figures 1a et 1b pour l'œil droit, et aux figures 2a et 2b pour l'œil gauche. Elles présentent l'état de la macula à la suite du traitement par des compositions conformes à l'invention (période N° 9, figure 1b OD et figure 2b OG) comparé à celui existant avant traitement (figure 1a OD et figure 2a OG). Les vues sont prises selon un même plan (au niveau de la fovéa) pour permettre la comparaison.

La formulation 5, administrée sous forme d'un traitement par cycles trimestriels avec prise pendant 2 mois et arrêt pendant 1 mois, se confirme être la plus efficace, tant pour la prévention et le traitement des drusen dans la MLA et la DMLA, que pour la préservation ou l'amélioration d'une acuité visuelle. Ce protocole respecte les équilibres biologiques, comme le montrent les résultats d'analyses.

## Revendications

1. Composition comprenant une préparation de Desmodium et un sel de chrome trivalent.

2. Composition selon la revendication 1, ***caractérisée en ce que*** la préparation de Desmodium est une poudre *Desmodium adscendens* ou de *Desmodium gangeticum et* obtenue par broyage des feuilles, des tiges ou des parties aériennes de la plante.

3. Composition selon l'une des revendications 1 ou 2, ***caractérisée en ce que*** le sel de chrome est choisi parmi le chlorure de chrome, le nicotinate de chrome ou le picolinate de chrome.

4. Composition selon l'une des revendications précédentes, *caractérisée en* ce *qu*'elle comprend 0,2 mg à 1,2 mg de chrome III par gramme de Desmodium, et de préférence de 0,25 mg à 0,5 mg de chrome III par gramme de Desmodium.

5. Composition selon l'une des revendications précédentes, *caractérisée en ce qu'*elle comprend de 30% à 100% de préparation de Desmodium et sel de chrome en masse par rapport à la masse totale de la composition.

6. Composition selon l'une des revendications précédentes, *caractérisée en ce qu*'elle comprend en outre au moins une vitamine choisie parmi la vitamine C, la vitamine E, la vitamine B1 , la vitamine B2, la vitamine B3, la vitamine B4, la vitamine B6, la vitamine B8, la vitamine B9 et la vitamine B12.

7. Composition selon l'une des revendications précédentes, *caractérisée en ce qu*'elle comprend en outre au moins un pigment choisi parmi la lutéine et la zéaxanthine.

8. Composition selon l'une des revendications précédentes, *caractérisée en ce qu*'elle comprend en outre une source d'acides gras de type oméga-3, choisie parmi une préparation de *Schizochytrium,* une préparation d'*Odontella aurita,* une préparation de graines de lin, ou un mélange de celles-ci.

9. Composition selon l'une des revendications précédentes, *caractérisée en ce qu*'elle comprend de 30% à 75% de Desmodium et de sel de chrome, de 10% à 30% de vitamines, et de 0,5% à 2,5% de lutéine et zéaxanthine, et de 25% à 60% d'une préparation d'huile de *Schizochytrium ou Odontella,* en masse par rapport à la masse totale de la composition.

10. Composition selon la revendication précédente, ***caractérisée en ce qu'***elle comprend de 60% à 65% de Desmodium et de sel de chrome, de 15% à 18% de vitamines, de 0,75% à 1,5% de lutéine et zéaxanthine, et de 30% à 40% d'une préparation d'huile de *Schizochytrium* ou d'*Odontella,* en masse par rapport à la masse totale de la composition.

11. Composition selon l'une des revendications précédentes, *caractérisée en ce qu*'elle comprend en outre une préparation sèche de d'*Aphanizomenon Flos-aquae* ou de Spiruline.

12. Composition selon l'une des revendications précédentes, *caractérisée en ce qu*'elle est formulée seule ou avec au moins un excipient ou additif, pour être administrée par voie orale sous forme de gélules, de comprimés, de capsules molles, ou sous forme liquide.

13. Composition selon l'une des revendications précédentes, pour son utilisation en tant que médicament, phyto-médicament, dispositif médical, complément alimentaire ou mélangée à un produit alimentaire.

14. Composition selon l'une des revendications 1-12 pour son utilisation dans la prévention ou le traitement d'une pathologie dégénérative de la rétine choisie parmi la maculopathie liée à l'âge, la dégénérescence maculaire liée à l'âge sèche, et la dégénérescence maculaire liée à l'âge humide.

15. Composition pour son utilisation selon l'une des revendications 13 ou 14, ***caractérisée en ce que*** la composition est administrée de manière quotidienne sous la forme d'un ou de deux emballages-doses.

## Patentansprüche

1. Zusammensetzung, die eine Desmodium-Zubereitung und ein Salz von dreiwertigem Chrom umfasst.

2. Zusammensetzung nach Anspruch 1, ***dadurch gekennzeichnet, dass*** es sich bei der Desmodium-Zubereitung um ein Pulver von *Desmodium adscendens* oder von *Desmodium gangeticum* handelt, wobei sie erhalten wird, indem Blätter, Stiele oder oberirdische Teile der Pflanze zerkleinert werden.

3. Zusammensetzung nach einem der Ansprüche 1 oder 2, ***dadurch gekennzeichnet, dass*** das Chromsalz aus Chromchlorid, Chromnicotinat oder Chrompicolinat ausgewählt ist.

4. Zusammensetzung nach einem der vorhergehenden Ansprüche, ***dadurch gekennzeichnet, dass*** sie 0,2 mg bis 1,2 mg an Chrom III pro Gramm an Desmodium, und vorzugsweise 0,25 mg bis 0,5 mg an Chrom III pro Gramm an Desmodium, umfasst.

5. Zusammensetzung nach einem der vorhergehenden Ansprüche, ***dadurch gekennzeichnet, dass*** sie 30 % bis 100 % an Desmodium-Zubereitung und Chromsalz umfasst, nach Masse bezogen auf die Gesamtmasse der Zusammensetzung.

6. Zusammensetzung nach einem der vorhergehenden Ansprüche, ***dadurch gekennzeichnet, dass*** sie darüber hinaus mindestens ein Vitamin umfasst, welches aus Vitamin C, Vitamin E, Vitamin B1, Vitamin B2, Vitamin B3, Vitamin B4, Vitamin B6, Vitamin B8 (Biotin), Vitamin B9 und Vitamin B12 ausgewählt ist.

7. Zusammensetzung nach einem der vorhergehenden Ansprüche, ***dadurch gekennzeichnet, dass*** sie darüber hinaus mindestens ein Pigment umfasst, welches aus Lutein und Zeaxanthin ausgewählt ist.

8. Zusammensetzung nach einem der vorhergehenden Ansprüche, ***dadurch gekennzeichnet, dass*** sie darüber hinaus eine Quelle von Fettsäuren des Typs omega-3 umfasst, wobei diese aus einer Zubereitung von *Schizochytrium,* einer Zubereitung von *Odontella aurita,* einer Zubereitung von Leinsamen oder einer Mischung davon ausgewählt ist.

9. Zusammensetzung nach einem der vorhergehenden Ansprüche, ***dadurch gekennzeichnet, dass*** sie 30 % bis 75 % an Desmodium und an Chromsalz, 10 % bis 30 % an Vitaminen und 0,5 % bis 2,5 % an Lutein und Zeaxanthin sowie 25 % bis 60 % einer Zubereitung von *Schizochytrium- oder Odontella-*Öl umfasst, nach Masse bezogen auf die Gesamtmasse der Zusammensetzung.

10. Zusammensetzung nach dem vorhergehenden Anspruch, ***dadurch gekennzeichnet, dass*** sie 60 % bis 65 % an Desmodium und an Chromsalz, 15 % bis 18 % an Vitaminen und 0,75 % bis 1,5 % an Lutein und Zeaxanthin sowie 30 % bis 40 % einer Zubereitung von *Schizochytrium-* oder *Odontella-*Öl umfasst, nach Masse bezogen auf die Gesamtmasse der Zusammensetzung.

11. Zusammensetzung nach einem der vorhergehenden Ansprüche, ***dadurch gekennzeichnet, dass*** sie darüber hinaus eine trockene Zubereitung von *Aphanizomenon Flos-aquae* oder von Spirulin umfasst.

12. Zusammensetzung nach einem der vorhergehenden Ansprüche, ***dadurch gekennzeichnet, dass*** sie für sich genommen oder mit mindestens einem Hilfsstoff oder Zusatzstoff formuliert ist, um auf oralem Wege in Form von Gelkapseln, Tabletten, Weichkapseln oder in flüssiger Form verabreicht zu werden.

13. Zusammensetzung nach einem der vorhergehenden Ansprüche, für eine Verwendung derselben als Arzneimittel, pflanzliches Arzneimittel, medizinische Vorrichtung, Nahrungsergänzungsmittel oder in Mischung mit einem Lebensmittel.

14. Zusammensetzung nach einem der Ansprüche 1 bis 12, für eine Verwendung derselben beim Verhüten oder Behandeln einer degenerativen Erkrankung der Netzhaut, welche aus der altersbedingten Makulopathie, der trockenen altersbedingten Makuladegeneration und der feuchten altersbedingten Makuladegeneration ausgewählt ist.

15. Zusammensetzung zur Verwendung derselben nach einem der Ansprüche 13 oder 14, ***dadurch gekennzeichnet, dass*** die Zusammensetzung täglich in Form von einer oder zwei Einzeldosispackungen verabreicht wird.

## Claims

1. Composition comprising a preparation of Desmodium and a trivalent chromium salt.

2. Composition according to claim 1, **characterized in that** the Desmodium preparation is a powder of *Desmodium adscendens* or *Desmodium gangeticum* and is obtained by grinding the leaves, stems, or aerial parts of the plant.

3. Composition according to one of claims 1 or 2, **characterized in that** the chromium salt is selected from chromium chloride, chromium nicotinate, or chromium picolinate.

4. Composition according to one of the preceding claims, **characterized in that** it comprises 0.2 mg to 1.2 mg of chromium III per gram of Desmodium, and preferably from 0.25 mg to 0.5 mg of chromium III per gram of Desmodium.

5. Composition according to one of the preceding claims, **characterized in that** it comprises from 30% to 100% of preparation of Desmodium and chromium salt by mass with respect to the total mass of the composition.

6. Composition according to one of the preceding claims, **characterized in that** it further comprises at least one vitamin selected from vitamin C, vitamin E, vitamin B1, vitamin B2, vitamin B3, vitamin B4, vitamin B6, vitamin B7 (biotin), vitamin B9, and vitamin B12.

7. Composition according to one of the preceding claims, **characterized in that** it further comprises at least one pigment selected from lutein and zeaxanthin.

8. Composition according to one of the preceding claims, **characterized in that** it further comprises a source of fatty acids of omega-3 type, selected from a preparation of *Schizochytrium,* a preparation of *Odontella aurita,* a preparation of seeds flax, or a mixture thereof.

9. Composition according to one of the preceding claims, **characterized in that** it comprises from 30% to 75% of Desmodium and of chromium salt, from 10% to 30% of vitamins, and from 0.5% to 2.5% of lutein and zeaxanthin, and from 25% to 60% of a preparation of *Schizochytrium* or *Odontella* oil, by mass relative to the total mass of the composition.

10. Composition according to the preceding claim, **characterized in that** it comprises from 60% to 65% of Desmodium and chromium salt, from 15% to 18% of vitamins, from 0.75% to 1.5% of lutein and zeaxanthin, and from 30% to 40% of a *Schizochytrium* or *Odontella* oil preparation, by mass with respect to the total mass of the composition.

11. Composition according to one of the preceding claims, **characterized in that** it further comprises a dry preparation of *Aphanizomenon Flos-aquae* or Spirulina.

12. Composition according to one of the preceding claims, **characterized in that** it is formulated alone or with at least one excipient or additive, to be administered orally as capsules, tablets, softgels, or in liquid form.

13. Composition according to one of the preceding claims, for its use as a medication, phyto-medication, medical device, food supplement, or mixed with a food product.

14. Composition according to one of claims 1-12, for use in the prevention or treatment of a degenerative retinal pathology selected from age-related maculopathy, dry age-related macular degeneration, and wet age-related macular degeneration.

15. Composition for use according to one of claims 13 or 14, **characterized in that** the composition is administered on a daily basis in the form of one or two dose packs.
